# EUROPEAN PATENT SPECIFICATION

(11) **EP 0 653 492 B1**
(45) Date of publication and mention of the grant of the patent: **27.09.2000**
(21) Application number: 94308364.2
(22) Date of filing: 11.11.1994
(51) Int. Cl.: C12Q 1/24, C12Q 1/34, C12Q 1/06, G01N 33/569, C12Q 1/68

(54) **Process for obtaining individual microorganisms, and applications of that process**
Verfahren zur Gewinnung von einzelnen Mikroorganismen und Anwendungen dieses Verfahrens
Procédé pour l'obtention de microorganismes individuels et applications du procédé

(30) Priority: 15.11.1993 JP 30709693; 15.11.1993 JP 28471993; 15.11.1993 JP 30707993; 15.11.1993 JP 30703493; 16.12.1993 JP 31658193; 18.01.1994 JP 334994
(43) Date of publication of application: 17.05.1995
(73) Proprietor: CANON KABUSHIKI KAISHA, Tokyo (JP)
(72) Inventor: Kuriyama, Akira, c/o CANON K.K., Tokyo (JP); Sugawa, Etsuko, c/o CANON K.K., Tokyo (JP); Yano, Tetsuya, c/o CANON K.K., Tokyo (JP); Miyazaki, Takeshi, c/o CANON K.K., Tokyo (JP); Kawaguchi, Masahiro, c/o CANON K.K., Tokyo (JP)
(74) Representative: Beresford, Keith Denis Lewis

(56) References cited:
- EP-A- 0 266 881
- EP-A- 0 395 355
- WO-A-91/04318
- WO-A-92/18643
- WO-A-94/16099
- GB-A- 2 266 153

## Description

### FIELD OF THE INVENTION

The present invention relates to a process for separating into individuals microorganism populations adhered to a carrier or to other microorganisms. The invention further comprises collecting separated microorganisms either as a precipitate or suspended in a liquid, and performing various operations on these microorganisms. Such operations can include counting the total microorganisms present, estimating the population of a particular type of microorganism as a proportion of the total, and separating DNA material from the individual microorganisms.

### RELATED ART

There is a demand for separation of individual microorganisms from suspensions of soil, from activated sludge in sewage plants, from waste water disposal tanks or mud at the bottom of a river, lake or the sea. In each such environment there is a large population of microorganisms of different kinds, and there is a requirement to be able to study these organisms and to extract the nucleic acid contained in them not only for fundamental studies in genetic engineering but also for their utility in various applied technological fields.

The process which has been mainly used up until now to bring about microorganism separation has involved forming a suspension of the microorganism-containing material in a suitable buffer solution and painting the suspension onto an agar culture medium containing appropriate growth materials. The plate is cultured and after growth has occurred, a colony of the microorganism which it is intended to study is selected, further cultured to a high concentration and then precipitated by centrifugal separation and collected. However, even though there may be a useful microorganism present in a suspension being sampled, it may not be successfully recovered by the above procedure. Only a small proportion of the microorganisms present in the suspension is actually collected for smearing onto the plate and 99 - 99.9% of them remain attached to the soil or activated sludge or mud in the suspension being sampled. Hyperplasia on the agar culture medium used makes it difficult to separate colonies of microorganisms of individual types. Furthermore, the culturing conditions may not be appropriate for the growth of all of the microorganisms present in the original sample, and it is not known in advance what culturing conditions would suit every microorganism of interest.

In the sampling of activated sludge in water sludge disposal tanks, the necessary culture conditions are relatively well established. However, culturing is likely to change the populations of the different kinds of microorganism in the culture medium compared to those present in the original tank because of subtle differences in the actual conditions in the tank and the conditions in the laboratory culture medium. The result is that a microorganism which is present in high population in the activated sludge might not be present in high population in the cultured sample.

In order to overcome this problem, microorganisms have been separated from a suspension of soil, activated sludge or mud at the bottom of the tank directly without any intermediate culturing process. However, such a microorganism-containing liquid is different from the liquid culture medium in which the microorganism is cultured and it contains various solids in addition to the microorganisms of interest. In consequence, collection of microorganisms from the liquid directly by filtration or centrifugal sedimentation is difficult. Furthermore, where a microorganism produces secretions that either adhere it to a solid substance or adhere individual microorganisms together to form a flock, it is difficult to collect individual microorganisms by filtration or by centrifugal sedimentation. Up until now, separation has been carried out by strongly stirring the suspension to bring about physical separation of the microorganism from the solid substances. The suspension has been agitated by a reciprocating shaking machine, a blender, a homogenizer or an ultrasonic dispersing machine. It is not clear, however, to what extent microorganisms recovered by this process are representative of those present in the original sample. The longer and the more violently the suspension is agitated, the more microorganisms are likely to separate. However, if the agitation is too strong, there is a risk that the microorganisms may be broken up, especially in the case of yeast, small algae or protozoa which are relatively large microorganisms and are fragile.

Furthermore, although some microorganisms can be separated from solid materials, recovery of the microorganisms by centrifugal separation is difficult. It is necessary to rely on slight differences in the centrifugation rates between the microorganism and other solid substances present in the test sample. Careful selection of the centrifuging conditions is required which makes the task more difficult. Collecting the microorganisms by the alternative route of suction filtration requires frequent changes of filter paper because solid material readily block it, so that this process is also complicated to carry out and is inefficient. In summary, existing processes do not effectively collect all kinds of microorganism present, do not provide a recovered sample in which the population of the various microorganisms is the same as in the material being studied, are liable to kill the more fragile microorganisms as a result of the steps necessary for microorganism recovery, and may fail to collect useful microorganisms present in the soil for whom the necessary conditions for a culture to thrive are not present. There is a particular problem in the separation of microorganisms attached to solid material such as soil or which have formed flock because they cannot be separated effectively by filtration or centrifugal separation.

There is a further requirement to be able to collect DNA and other nucleic acids from microorganisms recovered from the samples. The quality and/or purity of the DNA collected is very important in subsequent treatment, e.g. digestion using a restriction enzyme, the polymerase chain reaction, or hybridization. In the case of soil microorganisms, organic materials such as humus may be adhered to a carrier such as a soil particle on which the microorganism being studied is also present, and the recovered microorganisms may contain excessive amounts of these organic contaminants. Therefore the recovered material requires further refinement using the cesium chloride equilibrium density-gradient method or using a centrifugation refining process, or by using gel filtration e.g. hydroxyapatite column chromotography. These refinement methods are described in "Molecular Cloning, A LABORATORY MANUAL, SECOND EDITION", J. Sambrook et al, Cold Spring Harbour Laboratory Press, page 1.40 - page 1.48 and "Seibutsu Kagaku Jikkenhou 11, Geru Rokaho (Biochemical Experimentation 11, Gel Filtration)" written by Kensuke Shimura et al. Gakkai Shuppan Center (Society Press Center), page 181 - 195. However, the above processes involve lengthy and complicated tasks e.g. centrifugal separation over a period 24-48 hours using an ultra-centrifuge. They may necessitate the use of ethidium bromide which is a carcinogen, and the collecting efficiency of DNA is low.

In addition to the need to separate and collect particular microorganisms, it is also important to be able to count the number of individuals present in a suspension so that the state of the microorganisms in a bioreactor or biological system can be evaluated. A popular process for counting the number of microorganisms directly has been developed by P.C.T. Jones and J.E. Molison. It involves mixing the suspension to be studied with molten agar, stirring it and fixing it as film on a hemocytometer. The film is then dyed or stained and counted using a microscope. The Jones-Molison process is described in "Dojo Biseibutsujikkenho (Soil Microorganism Experimentation)", edited by Dojo Biseibutsukenkyukai (Society for the Study of Soil Microorganisms), published by Yokensha, page 143-154. This process has the advantage that numbers of microorganisms can be counted even though it is not known what conditions would be needed to culture them. However, the results obtained will lack validity if the microorganisms are insufficiently separated and only a fraction of them is collected. In the case of soil suspensions, activated sludge or mud from the bottom of a river, lake or sea, or liquid from a reactor including a carrier on which the microorganisms grow, or in the case where the microorganisms adhere to solid materials or to one another to form a flock, accurate counting is impossible. Furthermore, where there are many microparticles in the sampled liquid it is impossible to make a useful agar film on a hemocytometer.

In instances where the conditions for culturing the target microorganisms are known, dilution plate counting can be used. The suspension to be counted is suitably diluted, the diluted liquid is painted onto an agar culture medium, and the number of colonies of the target microorganism which have grown are counted. In this process, the growth phase can be carried out using a petri dish, and a thermoregulator, and it is easier than the Jones-Molison method where counting is done directly through a microscope. However, this process has the problem that it may take several weeks to carry out the culturing step, and it is only available where suitable culturing conditions for the target microorganism are known. Furthermore, all species of microorganism for which the culturing conditions are suitable will produce colonies so that this method is insensitive to microorganisms of different kinds. For most microorganisms in the sample under test, the conditions for growth on an agar culture medium are not known so that 99 - 99.9% of the microorganisms present will not be separated and detected. Furthermore, this method gives no information concerning the state of distribution of a particular organism in the microorganic system since it does not count all the microorganisms present.

Japanese Laid Open Patent Application JP-A2-170053 discloses a process for rapidly determining the presence of a test microorganism in a sample. One portion of the sample is treated with a tagged antibody which can bind to the target microorganism if present in the sample. After reaction has taken place, fluorescence measurements are carried out, and appropriate statistical calculations are carried out. A second portion of the test sample is not reacted with the antibody, and appropriate spectroscopic measurements are carried out after which the measured data is again statistically analysed. The results of the two calculations are compared to determine whether or not the target microorganism is present. This process has the advantage that the target microorganism can be detected easily and without the need to carry out a microorganism culturing step. However, it is impossible to measure the total number of microorganisms in the sample or the distribution of the target microorganism in the sample because this process only detects whether or not the target microorganism is present. One of the reasons why this process does not enable microorganisms to be counted is that there is a contribution to the fluorescence signal from cohered flocks of microorganisms. Furthermore there is a need for a control sample to be prepared so that the desired information cannot be obtained in a single measurement.

It is of considerable technological significance to be able to measure the number of microorganisms present in a sample as a proportion of the total number of microorganisms growing in that sample in order to determine the effect of different conditions on the population of a target microorganism. For example it is important to be able to study the activity of a target microorganism in a bioreactor where it is in symbiosis with a plurality of other types of species of microorganism. It may also be desired to study the change in distribution of enterobacteracease as a result of pharmacological action. It is also a particular advantage to be able to determine the population distribution quickly and correctly using only a single measurement.

### SUMMARY OF THE INVENTION

This invention is based on the realisation that microorganisms which are adhered to solid substances or cohered to each other to form a flock can be separated into free individuals without any culturing process by a treatment which decomposes the natural material which binds the microorganism to the solid substance or to the other microorganisms, and thereby the individual microorganisms can be separated and collected from liquid suspension at high yield and in high purity.

In one aspect there is provided a process for separating individual microorganisms which are adhered to a carrier or to other microorganisms, which comprises:
providing a microorganic system in which there are present individual microorganisms adhered to a carrier or to other individual microorganisms; and
decomposing by means of an enzyme the natural material which adheres the individual microorganism to the carrier or to the other microorganisms so as to separate the individual microorganisms.

Other preferred aspects of the invention are claimed in the accompanying claims to which attention is hereby directed.

### BRIEF DESCRIPTION OF THE DRAWINGS

How the invention may be put into effect will now be described by reference to the accompanying drawings in which:

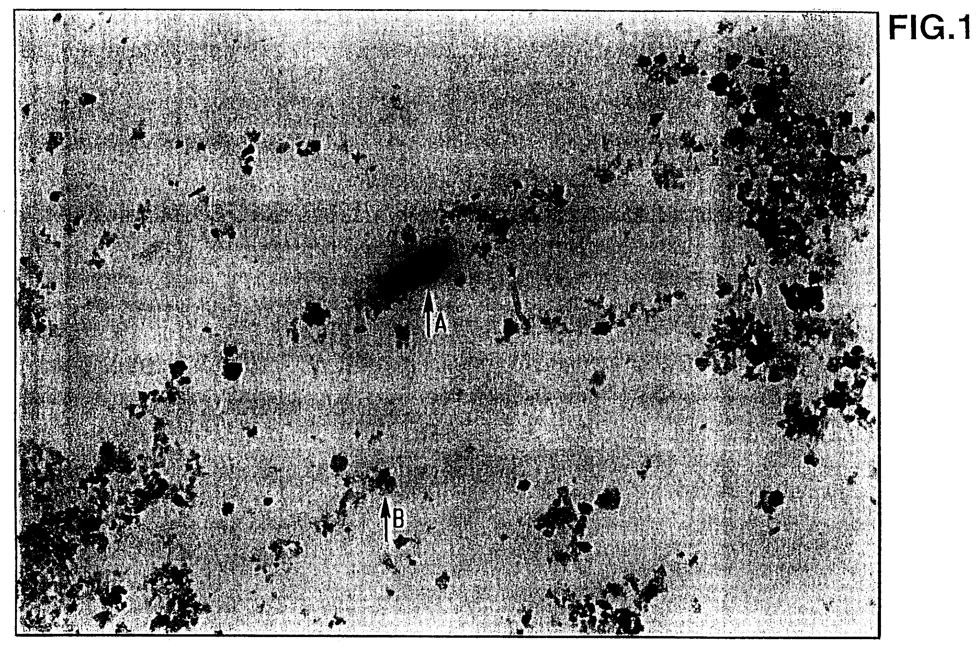

Fig. 1 is a micrograph (times 2000) showing microorganisms and other particles in a supernatant liquor obtained by the process of Example 7 below;

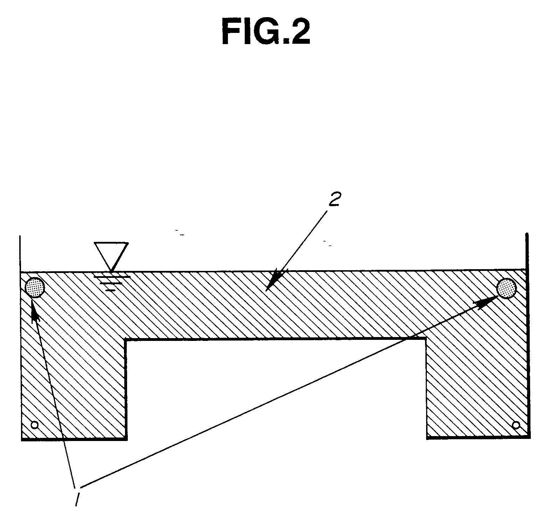

Fig. 2 is a diagrammatic view in vertical section showing an electrophoresis tank used in Example 9 below;

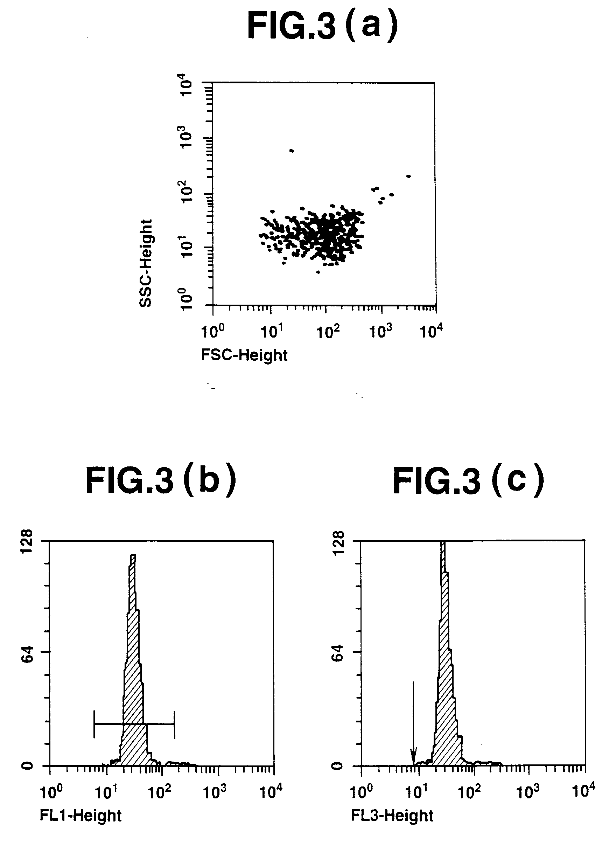

Figs. 3(a), (b) and (c) are graphs showing the results obtained using a through-flow fluorescence spectrometer in Example 10 of the present invention. Fig. 3(a) is a dot plot showing forward scattering (FSC) and side scattering (SSC) fluorescence. Fig. 3(b) is a fluorescence histogram showing the range of measured signal strength in a channel FL1 (530 ± 15 nm). Fig. 3(c) is another histogram of measured fluorescence signal strength in a channel FL3 (650 nm) with the arrow representing a trigger level; and

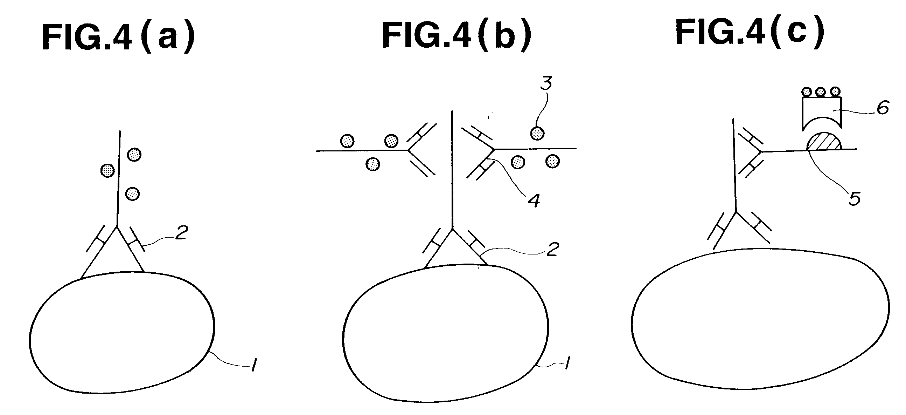

Figs. 4(a), (b) and (c) are diagrams showing the attachment of a tagged antibody to a target microorganism in a counting process according to the present invention. Fig. 4(a) shows the attachment of a fluorescent tagged antibody to the microorganism. Fig. 4(b) shows the indirect attachment of a fluorescent tagged secondary antibody to the target microorganism. Fig. 4(c) shows the attachment of a fluorescent dye to a particular microorganism using a biotinated second antibody and fluorescent tagged avidin.

### DETAILED DESCRIPTION OF THE INVENTION

### Microorganisms and their enzvme treatment

The process of the invention is applicable to a wide range of microorganisms having different growth habits, including bacteria, actinomycete, yeast, moulds, fungi, micro-algae and protozoa, and is applicable to microorganisms existing in or isolated from nature and microorganisms used in biochemical processes or obtained by the application of biotechnology. It is applicable both to microorganisms which have become adhered to a carrier, and to microorganisms living in soil, those living in activated sludge of waste water disposal tanks, and living in mud at the bottom of rivers, lakes or seas.

In these environments the living microorganisms are generally attached to particles of soil or other carrier particles. The invention is also applicable to microscopic samples taken from a living or dead animal or plant, and where the microorganisms commonly cohere to each other to form a flock. It is also applicable to microorganisms which are both adhered to one another to form a flock and attached to a carrier. In bioreactors, microorganisms are commonly attached to a solid carrier, and such microorganisms can also be separated by the method of the invention. The method is applicable to the treatment of living microorganisms and after the separation process has been carried out the individual microorganisms remain alive in suspension.

Many living microorganisms secrete insoluble organic polymers by which they are adhered to a substrate or to one another to form a flock. Because of this natural adhesive it is difficult to separate individual organisms by physical means e.g. using a blender, and separation using chemical solvent is likely to cause the microorganism to break up. This invention is based on the use of an enzyme to digest the natural adhesive since this process does not lead up to break up of the organism and it has been found that the polymer can be decomposed and removed effectively.

The decomposition enzyme can be added to the original microorganic system, but it is preferred to do so under conditions in which the population of microorganisms does not increase or decrease and the proportion of the individual microorganisms present does not change. The enzymes used and the operational procedures will depend upon the sample being studied and the microorganism(s) present. One form of the separation process usable in the invention involves the following steps:
(a) A sample gathered from a microorganic system under test is suspended in a buffer regulated to the best working pH of the enzyme to be used so as to provide a suspension of the microorganisms and any associated carrier particles. In the case of cellulase the optimum pH range is 4-6;
(b) Adjusting the concentration of solids to 5-20 wt.%;
(c) Adding an enzyme which is effective to decompose the natural adhesive and liberate the individual particles;
(d) Filtering and removing coarse suspended solids or any floating materials;
(e) Maintaining the filtrate for about 2-16 hours under agitation at the working temperature of the enzyme, preferably about 40 to 50°C in the case of cellulase; and
(f) Centrifuging the diluted microorganic system e.g. at 3000 to 5000 rpm for about 10 seconds to 1 minute to precipitate microparticles of soil etc present and then collecting a supernatant fluid containing the microorganisms.

If the reaction time of the enzyme is too short, the natural adhesive will be insufficiently digested and the individual microorganisms will be incompletely released. The population of microorganisms may increase or may decrease, and the individual microorganisms may be destroyed. Selection of the appropriate reaction condition is a matter of trial and error in each individual case. After the reaction and separation of the microorganisms is complete, it is desirable to proceed immediately to the next process, e.g. counting the individual microorganisms present.

The enzyme system which may be used in the process of the above invention may be a single enzyme or a mixture of enzymes. These may be selected from polysaccharide-digesting enzymes such as cellulase, hemicellulase, glucuronidase, amylase, glucanase and xylanase and protein-digesting enzymes, e.g. protease, collaginase, and enzymes for digesting pectin, e.g. pectinase and pectin transeliminase. The most preferred enzymes are cellulase, hemicellulase, glucuronidase, amylase, protease and pectinase. One particularly effective mixture of enzymes comprises cellulase as the main ingredient together zylanase and optionally one or more further enzymes. A further preferred mixture of enzymes comprises cellulase together with glucuronidase or cellulase together with protease. The amount of enzyme to be added to the sample will differ depending upon the nature of the enzyme, the species of the microorganisms believed to be present and/or the amount of living microorganisms to be present. However, in the case of cellulase the amount to be added to the diluted suspension is usually 1000 U/ml or above of cellulase activity, preferably 10000 U/ml or above. Where the enzyme is protease, the amount to be added to the diluted suspension normally provides 100 U/ml or above of protease activity, preferably 1000 U/ml or above. The two enzymes may be used as a mixture, as explained above. In the case of glucuronidase, the amount present will normally provide 10 mg/ml or above of the crude enzyme, preferably 100 mg/ml or above. If the amount of the enzyme is insufficient, the individual microorganisms will be incompletely liberated by decomposition of the natural adhesive. There is no upper limit on the amount of enzyme, but excessive amounts of enzyme do not lead to a corresponding increase in the degree of decomposition.

It has been difficult up to now to obtain an accurate count of the bacteria present soil. The present process, however, is believed to produce a separation into free individual microorganisms of about 10 to 90% by number of all those present in the soil. The process enables all the species of microorganism which are present in the sample to be collected, so that in principle the proportion of the different species of microorganism in the sample should not be changed from those present in the original. Microorganisms which have been cohered as a flock can be separated into individuals according to the invention, which facilitates the isolation of a target species from the mixture of microorganisms by only one process of screening and to culture of it under conditions appropriate to that microorganism.

### Processes for collecting and refinement of the microorganisms

Two main mechanisms for collecting and refining microorganisms will now be described. One of them is based on centrifugation, and the other is based on subjecting the microorganism-containing solution to a voltage applied between electrodes and collecting the microorganisms from adjacent one of the electrodes.

The centrifugation process is based on the concept of suspending the microorganisms in a medium having an identical or closely similar specific gravity. Other suspended solids will normally have specific gravities which are either greater or less than that of the microorganism and the surrounding medium, so that the effects of centrifugation will be to cause them to separate either by flotation or by sinking. The inventors have found that the specific gravities of microorganisms in soil, activated sludge, mud from rivers, lakes and seas is in the range 1.2 - 1.3, whereas solids which are also present may have two or more different specific gravities which lie outside the aforesaid ranges. It is therefore preferred to adjust the specific gravity of the suspension to lie in the range 1.2 - 1.5, preferably about 1.3. If the specific gravity of the medium lies outside this range, the effectiveness and reliability of the microorganism separation is reduced. Centrifugation is normally such as to apply a force of 200,000G or more, because although solid particles normally have a higher specific gravity than that of the solution, their sedimentation velocity is low because the particles may be of submicron size.

The solution in which the materials are suspended for centrifugation may contain a wide range of solutes, other than strong acids and strong alkali, but weak acids for example citric acid may be acceptable. Representative materials include sucrose, cesium chloride, cesium sulphate and Ficoll (Registered Trade Mark). Sucrose has the advantage that it is inexpensive and it does not change the pH of the material in which it is dissolved. Cesium chloride has the advantage that it is dense and can easily be made up into a highly concentrated solution.

A representative centrifugation process involves the following specific steps. A sample containing 4 ml or more of material is introduced into a centrifuge tube made of polyallomer, and the tube is centrifuged at 10,000 - 50,000 rpm, preferably 50,000 rpm for 1 to 10 hours, preferably 1 to 2 hours to bring about precipitation of compounds having high specific weight. The speed of revolution and the time are preferably selected to be the minimum for effective removal of the microparticles, and the conditions required in any instance can be determined by routine trial. Solutions of sucrose can be viscous so that they may need a slightly longer centrifugation period, and the temperature at which centrifugation is carried out is room temperature or above for optimum purity of the refined and collected microorganisms.

After centrifugation has been completed, there will be many microparticles at the solid/liquid interface of the centrifuged sample which are prone to mix into the supernatant liquor when that liquor is sucked up into a pipette. Therefore it is preferable to collect the supernatant liquor from the upper layer carefully, and to stop the collection when the surface of the supernatant liquor reaches about 5 mm from the interface with the precipitated solids. The supernatant liquor has a cloudy appearance resulting from movement of the microorganisms selectively into it, and the solute used to adjust the specific weight together with some organic compounds and residues of living cells may also be present in it.

For further refinement of the microorganisms collected the supernatant liquor is diluted to 5-10 times, e.g. by means of distilled water, to adjust the density to about 1.05 g/ml. The supernatant liquor is then centrifuged e.g. at about 5000 - 15000 rpm for 10 - 30 minutes to precipitate the microorganisms. The precipitate may be resuspended and recentrifuged several times to bring about further purification as required.

Where only a few microorganisms are present, e.g. 10⁴ - 10⁵, it is preferable to dissolve polyethylene glycol (PEG) to give a concentration of 20% before centrifugation in order to increase the collection efficiency. When the microorganisms have been collected, if it is desired to count them, they may be diluted with an appropriate volume of distilled water.

An alternative collection and purification process involves placing electrodes in the microorganism suspension and applying a voltage between the electrodes with the result that the microorganism will move under the influence of the applied electric field. Such a process involves the steps of diluting the enzyme treated suspension, described above, with an appropriate volume of distilled water and pouring it into a tank containing the electrodes; applying a DC voltage between the electrodes to bring about microorganism movement towards one or both of the electrodes; and allowing solids in the suspension to precipitate under the influence of gravity while the DC voltage is maintained so as to bring about separation of the microorganisms, and subsequently collecting the microorganisms.

In this variant of the collection process the concentration of microorganisms in the suspension to be separated by the electric field, the operating conditions such as the applied voltage and the like are selected depending on the characteristics of the microorganism to be collected. For example, the applied voltage may have a value of several tens to several hundred volts, preferably 20 - 150 volts for a period of several hours. The electrodes are preferably located at an upper part of a water tank whose depth is preferably about 1-10 cm. The soil particles present in the suspension precipitate under the influence of gravity so that a separation between the microorganism and the soil particles is easy to obtain. The volume of the tank may preferably be about several hundred ml not including the sample volume and is maintained at a generally constant temperature during the electrophoresis operation.

Microorganisms collected by electrophoresis may be further purified by centrifugation with appropriate resuspension or dilution in distilled water and appropriate adjustment of the specific gravity of the suspension, and the precipitated microorganisms can be collected relatively easily.

### Procedure for counting the number of individual microorganisms

The separated microorganisms may be counted by the dyeing method or by the dilution plate counting mechanism. In the dyeing method, microorganism-containing liquid collected and refined by the procedure described above is concentrated as required to give, for example, a concentration of about 10⁷ bacteria/ml. Then the liquid suspension of bacteria is treated with an equal volume of formalin (about 4%) in order to fix the microorganisms. The microorganisms are dyed or stained with a dye which is fluoroescent under ultraviolet light, and impurities are removed as necessary. The liquor containing the dyed microorganisms is dropped into a counting chamber and observed by a fluorescence microscope which enables the number of individual microorganisms to be counted. A variety of dyes may be used to stain the microorganism for counting.

Prior to counting, the sample preferaby has particles of size 100 µm or more removed therefrom by a filter, and also particles of lower specific weight than the microorganisms removed therefrom. The sample is preferably diluted or concentrated to bring the microorganism concentration into the range of 1 x 10² - 1 x 10⁹ cells/ml. To facilitate the counting process, a fluorescent dye is bonded selectively to a target microorganism, or a fluorescent compound is bonded to all the microorganisms unspecifically. Specific bonding of a dye to a microorganism can be achieved using an antibody which bonds selectively to an antigen of the target microorganism and which is a carrier for the dye. Monoclonal or polyclonal antibodies may be used, but monoclonal antibodies are preferred because they have greater selectivity. Microorganisms generally have a plurality of epitopes, so that it is possible to use a mixture of monoclonal antibodies in order to decrease the antiselectivity.

In the present labelling processes, firstly an antibody A is attached to a target microorganism, after which if necessary an antibody B having a specificity to the antibody A is attached to the antibody A. If the antibody A is labelled it is not necessary to use the antibody B, and where the antibody B is labelled, it is not necessary to label the antibody A but the second stage reaction is required. The antibody B is normally referred to as a secondary antibody. An example in which a fluorescent dye is carried by a specific microorganism by directly reacting with a fluorescent labelled antibody is shown in Figure A. Figure 4B is an example where a fluorescent labelled secondary antibody is attached to a target microorganism, and Figure 4C is an example in which a bitonated secondary antibody and fluorescent tagged avidin are attached indirectly to the target microorganism.

The monoclonal antibodies may be obtained by conventional methods. An animal is subjected to microorganisms to give rise to antibodies depending on the actigen of the microorganism. Immune cells making the required antibody recovered from the organism are fused with other cells to make an immortal cell line. There may also be employed a process or processes in which a gene is expressed which codes for the desired antibody.

Dyes for labelling the antibody may be of known fluorescent types. Examples include fluorescin isothiocyanate (FITC), rhodamine X isothiocyanate (XRITC), tetramethylrhodamine isothiocyanate (TRITC), phycoerythrin (PE), Texas Red, chlorotetracycline, a cynanin dye, a merocyanin dye such as merocyanin 540, dansyl chloride eosim, an oxonal dye, fluorescamine, anzil aziridine, 5-iodacetoamidefluorescin, N-(1-anilinonaphthyl-4)-maleimide and eosin-5-iodoacetonamido. FITC, XRITC, TRITC, PE and Texas Red are preferred in view of the wavelength of their maximum fluorescence and/or their fluorescent strength. The dye and its conditions of use may be selected depending on its absorption characteristics and fluorescent wavelength and also depending upon the light which is available for irradiation of the dye.

Antibodies may be tagged with the fluorescent compounds by known processes. For example, where an antibody is to be tagged with FITC a solution containing the antibody is adjusted to pH 8 - pH 9 FITC powder is added to the solution and reaction is allowed to take place over a period of four hours at 5°C, after which unreacted fluorescent dye is removed by gel filtration to give the desired fluorescent labelled antibody. The coupling ratio of the fluorescent dye to the antibody (F/P ratio) may be 0.1-20, preferably 1-6. For non-specific fluorescence labelling of microorganisms, the fluorescent dye used may be any compound which stains the microorganism but does not have specificity for other particles present. One impossible staining method is for the compound to bind to a protein located at the surface of the microorganism. Such staining dyes include 4,4-diisothiocyano-2,2'-disulfonic acid stilbene(DIDS), hematoporphryn, etc. However, fluorescent compounds are preferred which form a bond to the nucleic acid present in the microorganism, and such fluorescent compounds may be used singly or in a mixture of two or more. Preferred such compounds include thiazole orange, proflavin eripsin, donomycin (doxorobicin hydrochloride), acronol (phloxine FES), 3,3'-dimethylthiocarbocyanin, 3,3'-diethylthiocarbocyanin, 3,3'-diethyl-9-methylthiocarbocyanin bromide, 2-[γ-1'-ethyl-4',5'-benzothiazolyliden propenyl]-3-ethyl-4,5-benzothiazolum iodide, astrazon red 6B, basic violet 16, 2-(p-dimethylaminostyryl)-3-ethyl-4,5-benzothiazoliumiodide, 2,4-bis-(p-dimethylaminostyryl-1-ethyl) pyridinium iodide, 2,6-bis-(p-dimethylaminostyryl)-1-ethyl-pyridinium iodide, astrazon orange R, auramine O, acridine orange, ethydium bromide, propidium iodide, neutral red, basic yellow 11, acridine red 3B, rodamine S, thiazole orange, rodamine 6G, rodamine B, rodamine 19 perchlorate, rodamine 123, eosin Y, cyanothin, cresyl fast violet, durol red and the like. Amongst these, ethydium bromide propidium iodide, acridine orange and thiazole orange are particularly preferred because their fluorescent strength increases when they bond to the nucleic acid of a microorganism. Those dyes may be selected which have a desired absorption wavelength and fluorescent wavelength depending upon the light which is to be used to irradiate them.

In order to permit measurement of fluorescent light from a fluorescent dye used for tagging a target antibody, and also fluorescent light from a compound which is bond non-specifically to the microorganisms, the wavelength distributions of the dye and the fluorescent compound are preferably arranged to have minimal overlap. If there is a significant overlap of their fluorescence bands, it is difficult to filter unwanted fluorescent light and the S/N ratio is decreased. Thus, the fluorescent dye to be used preferably has a maximum fluorescent wavelength (λ max) which differs from that of the fluorescent compound by 20 nm or above, preferably 30 nm or above.

The two steps in which firstly a target microorganism is selectively labelled by means of a fluorescent tagged antibody and the step in which a fluorescent compound is non-specifically bonded to the microorganisms present may be carried out in any desired order, or they may be carried out at the same time. Thus, both the fluorescent tagged antibody and the fluorescent dye may be added at the same time to a suspension containing the organisms and allowed to react. The amount of fluorescent tagged antibody to be added is adjusted depending on the amount and type of the target microorganism and the intended reaction time. However, it is preferred that the antibody is added in large amount, for example 0.01-2 mg/ml, preferably 0.05-0.8 mg/ml. The amount of the fluorescent compound is also adjusted depending on the concentration of microorganisms in the sample and the intended reaction time. Again, the fluorescent compound is preferably added in large amount, for example in the range of 5-500 µg/ml, particularly 10-100 µg/ml. If the amount of fluorescent compound is too low, the number of microorganisms which can be counted is decreased, whereas if the amount is too great, the S/N ratio is deteriorated because background fluorescent light appears. For the purposes of the fluorescent antibody tagging and fluorescent dye staining steps, 2-4% of formalin solution and 90-100% ethanol or methanol can be added to the sample suspension in order to fix it. An appropriate amount of 0.05-2 M phosphate buffer solution or citrate buffer solution can be added to adjust the sample pH to a suitable value.

There will now be described a process for detection of a particular target microorganism amongst the whole population of microorganisms in the sample by quantative fluorescent counting. A suspension containing microorganisms tagged with the fluorescent dye and stained with the fluorescent compound is irradiated by light from an argon, helium, neon, semiconductor or SHG-YAG laser, and the fluorescent light emitted by the dye and the compound are measured. The fluorescent light and the fluorescent compound are selected so that their wavelengths match. Photometry using light having two different wavelengths or three different wavelengths by using two or more different light sources having different wavelength distributions are preferably used in order to increase the sensitivity of the measurement or the number of different microorganisms that can be counted using antibodies having respective different fluorescence properties.

The generation and detection of the fluorescent light may be carried out using a batch-type photometer in which the suspension to be sampled is placed in an optical cell. Separate calibration curves are made relating to the amount of fluorescent light from the target microorganism and the amount of fluorescent light from the whole population of microorganisms in relation to the concentration of the microorganism in question. Then measurement of the strength of fluorescent light gives directly the concentration of the target microorganism and the concentration of all the microorganisms present in the sample. Signals may be obtained measuring forward scattered light, side scattered light, forward fluorescent light and side scattering fluorescent light using a plurality of optical detectors.

However, for accurate counting of the number of microorganisms, it is preferred to cause the suspension being sampled to flow through an optical cell irradiated with light and to detect a signal from each particle and carry out statistical analysis of the detected signal in order to determine the number of organisms present. The counting of the target microorganism can be improved in accuracy by counting each signal in which both the fluorescent light from the dye and the fluorescent light from the staining compound are detected together. Apparatus capable of such a measurement is based on flow cytometry and is preferably used. The flow cytometer is an apparatus in which the particles are caused to flow one by one in a current of liquid past a sampling position in which they are irradiated with laser light or ultraviolet light to produce an optical reaction such as light scattering or fluorescence, and the size and/or form and/or characteristics of the particles are detected from the scattered or fluorescent light. In the present counting process, where two or more different fluorescent dyes are present, the fluorescent light is preferably filtered into discrete components corresponding to each fluorescence peak and detected individually by using a respective photodetector. Commercially available apparatus for carrying out the above measurement includes the FACS-CAN machine manufactured by Becton & Dickinson Co., Ltd., and the Epicsprofile 11 machine manufactured by Coulter. For example, in the case of the FACS-CAN machine, the fluorescent light from the sample is divided into three components which are FL1 (530 ± 15 nm), FL2 (585 ± 21 nm) and FL3 (650 nm or above). Three photodetectors are provided which each detect a respective one of the above three fluorescent light components. As the test liquid is flowed through the cell, the individual microorganisms produce counts in the various counting channels, and the relative intensities can be formed into a histogram which can be used to decide what signals in that channel shall be accepted. Then counts within the channels determine the number of microorganisms detected. Furthermore, a detector which is sensitive to forward scattering light and a detector which is sensitive to side scattered light can provide information about particle size etc., and the accuracy of the count can be improved by disregarding the signals from oversized particles.

Furthermore, where the flow cytometer is used for normal purposes, a trigger or gate is set for the forward scattered light and/or the side scattered light, the number of particles can be measured by detecting the light scattered from the particles. Signals whose strength is less than a threshold are eliminated by setting a trigger, and a gate is set so that only signals falling within a desired range of intensity are accepted. Furthermore, in the flow cytometry method, particles are caused to flow one by one past the sampling point.

If an aggregate of microorganisms is formed by the antibody, the count will not be accurate. Where such an aggregate is formed, the strength of the fluorescent light is increased so that the aggregate can be detected from the strength of the forward scattered light. However, the aggregate is preferably separated in advance because that facilitates the counting step. Such a separation may be carried out using vibrational energy e.g. from cavitation by means of a supersonic wave or shearing energy which is the result of stirring, and energy sufficiently strong to break up the microorganism is not required. Suitable supersonic wave treatment may be at 0.1-10W and 20 kHz over a period of 1-10 seconds. The fluorescence measurement can be carried out effectively in a flow cytometer using the following procedure. A sample is aspirated and introduced into a sheath flow cell and permitted to flow for an appropriate period, until a required volume of the sample has passed through the cell. The number of particles is counted while flow is in progress. However, in the case where the particles to be counted are microparticles having a diameter of 5µm or less, such as bacteria, or where there are significant impurities having the same size as the microorganisms to be measured, e.g. proteins, lipids, dust or other particles, it is difficult to count the number of microorganisms simply by setting a trigger based on the forward scattered light or side scattered light which are conventionally used because the non-microorganism impurities generate noise. However, the noise signal can be cut by setting the trigger or gate based on a fluorescent light signal emitted from a dye which bonds to the nucleic acid. Almost all the impurities will be free of nucleic acid or have a very low content of nucleic acid. For example, in the above-mentioned channel FL3 where the fluorescent signal is produced by all the microorganisms present, a signal whose fluorescent strength is below that of a trigger or threshold is disregarded because it will come from impurities other than microorganisms. Referring now to the signal in a channel such as FL1 which records fluorescent signals corresponding to fluorescent tagged antibodies attached to a target microorganism, where an incorrect fluorescent light signal is present in that channel because of fluorescent tagged antibody bonded to an impurity, that signal will not coincide with a signal in channel FL3 because the impurity will not have been stained with the fluorescent compound. Therefore, the work involved in data processing can be decreased and the time required can be reduced and both the total number of microorganisms present and the number of target microorganisms present can be counted readily by counting the signal set with a trigger or gate.

In order to set the trigger or gate, the sensitive wavelength is preferably adjusted to near the maximum fluorescence wavelength of the fluorescent compound which bonds to all of the microorganisms. For example, wavelengths of 10-60 nm to either side of the maximum fluorescent wavelength can be accepted. The level of the trigger or gate is then adjusted appropriately depending on the strength of the fluorescent light emitted by the fluorescent compound at the wavelength of the trigger or gate. However, it may be preferable to set the trigger level depending on the signal strength obtained by measuring a known microorganism tagged in advance with a fluorescent compound. The use of a gate set as described above enables signals coming from microorganisms to be distinguished from those coming from impurities.

Furthermore, a fluorescent signal which comes from a fluorescent dye which is specifically bonded to a target microorganism can be arranged to be accepted when there is also present a signal which appears through the trigger or gate of the channel for the fluorescent compound so that there will be little noise from impurities and the like when the number of the target microorganism is being counted. It is preferable to gate the fluorescent light from the dye to be within a desired range which can be selected so that almost all the signal based on the fluorscent dye is accepted, but other signals from impurities are rejected. For this purpose, the width of the gate is preferably adjusted depending upon the distribution of fluorscent light intensity emitted by a known microorganism tagged with a fluorescent compound. Furthermore, the above-described operation can be carried out even where there is a plurality of antibodies tagged with fluorescent dye which bond to a plurality of target microorganisms specifically, the bonding being either direct or indirect.

### Separation of nucleic acid from collected and refined microorganisms

Embodiments of the invention can provide suspensions of microorganisms which are sufficiently pure that it is possible to collect nucleic acid with a sufficient degree of purity that it can be digested by a restriction enzyme without the need for cesium chloride equilibrium density-gradiant centrifugation or gel filtration which have been indispensible up to now.

In the DNA extraction procedure, a precipitated microorganism obtained by the methods described above is treated with a surfactant such as SBS to destroy the microorganism, after which the DNA is extracted into the aqueous phase using phenol or formalin, nucleic acids are precipitated using ethanol to collect to microbial nucleic acid. If it is desired to recover microbial DNA from the suspension, it is preferably treated with RNase. If it is desired to obtain the RNA, the suspension may instead be treated with DNase.

Hereinbelow the present invention will be explained in more detail with reference to the examples.

### EXAMPLE 1

### Capacity of enzymes to liberate individual microorganisms from soil samples

10 g (wet weight: moisture content ratio of 81.4) of a soil sample was mixed with 15 ml of about 0.1 M-sodium phosphate buffer solution which was previously sterilised at two atmospheres, 120°C for 30 minutes. Disodium hydrogenphosphate and sodium dihydrogenphosphate were added to the mixture which was then stirred for 30 seconds to produce a soil suspension having a pH of 4.6. A 1 ml sample of this suspension containing 500 mg of suspended solid matter was introduced into a 5 ml test tube having a lid. The capacity of the cellulase produced by a filamentous fungus called Trichoderma viride to liberate individual microorganisms for the soil sample was then evaluated. A powdered enzyme whose main active ingredient is believed to be cellulase and which is available from Meiji Seika Kaisha Ltd. under the name Meiji Cellulase TP was dissolved in distilled water in a proportion of 20 mg of enzyme to 1 ml of distilled water, giving an activity of cellulase of 12000 units. The resulting solution was filtered through a filter having a diameter of 0.45 µm to remove residual suspended solids.

A 1 ml portion of this dissolved enzyme was added into the test tube which was then shaken for 2 hours at 40°C in a thermostat. Thereafter the tube was centrifuged at 5000 rpm for 30 seconds to precipitate soil particles, after which 1.5 ml of supernatant liquor was collected. This supernatant liquor was centrifuged at 15000 rpm for 5 minutes to form a precipitate of the microorganisms present, and the precipitated microorganisms were resuspended in 50 µl of distilled water. Formalin (4%) was added to this liquid to fix the microorganisms, after which ethidium bromide which emits a fluorescent light on irradiation with ultraviolet light was added to stain the microorganisms. The solution was left for one hour to permit any micro-soil particles remaining in the supernatant liquor to precipitate, after which the solution was suitably diluted e.g. 100 times and introduced dropwise into a hemocytometer. Observation of the liquid in the hemocytometer by means of a fluorescence microscope confirmed that the microorganisms present were not attached one to another but were floating freely without formation of flock, and the number of individual microorganisms present was counted. As a result it was confirmed that about 3 x 10⁸ of bacteria could be collected from 1 g (wet weight) of soil.

### EXAMPLES 2 TO 5

### Evaluation of other enzymes for their capacity to release microorganisms for soil

The following powdered enzymes were evaluated using the procedure described in Example 1 with the results indicated below.

| EXAMPLE NO. | ENZYME | COLLECTABLE BACTERIA |
|---|---|---|
| 2 | Glucuronidase | 9 x 10⁸ |
| 3 | Protease | 2 x 10⁸ |
| 4 | Cellulase + Amylase | 6 x 10⁸ |
| 5 | Cellulase + Protease | 1 x 10⁸ |

The glucuronidase used is available under the trade name Abalone Aceton powder from Sigma Chemical Company and was dissolved in a proportion of 100 mg of the enzyme to 1 ml of distilled water. The protease is available under the name Protease A Amano from Amano Pharmaceutical Co. Ltd. and was used in a proportion of 50 mg to 1 ml of distilled water giving a protease activity of 500 units. The amylase used in Example 4 is available under the trade name Termamyl from Novo Nordisk Bioindustry and a 500 µl sample of the filtered enzyme was used together with 20 mg of the cellulose acetate solution employed in Example 1. In Example 5, 20 mg portion of the cellulase solution used in Example 1 was employed together with 50 mg of protease which is available under the name Protease A. Amano from Amano Pharmaceutical Co. Ltd. In each of the above examples, after the described procedure had been carried out groups of bacteria were not detected, and the bacterial counts were made by a fluorescence microscope as previously described.

### COMPARATIVE EXAMPLE 1

### Repetition in the absence of enzyme

The procedure of Example 1 was repeated except that no enzymes were introduced into the test tube. Observation of the fixed and stained supernatant liquor by means of a fluorescence microscope showed that groups of bacteria were present and about 3 x 10⁷ of bacteria had been collected from 1 g (wet weight) of soil which is only 10% of the number observed in Example 1, 6% of the number observed in Example 2 and 15% of the number observed in Example 3.

### COMPARATIVE EXAMPLE 2

### Repetition with deactivated enzyme

The procedure of Example 1 was repeated except that prior to addition into the test tube the cellulase was deactivated by being maintained at a pressure of two atmospheres and at a temperature of 120°C for 30 minutes. Observation of the dyed suspension by means of a fluorescence microscope indicated the presence of groups of bacteria. It was estimated that about 4 x 10⁷ of bacteria were collected from a 1 g wet weight soil sample, but this was only 13% of the number of bacteria observed in Example 1 and 7% of the number observed in Example 2.

### EXAMPLE 6

### Isolation of microorganisms attached to a solid substrate

100 g of porous zeolite of diameter about 1 mm (trade name Rainbow Sand, manufactured by Sanko Kenso K.K.) were introduced into a bioreactor of volume about 300 ml which was then filled up with 200 ml of a culture liquid. The bioreactor was operated for about ten days, after which it was apparent that a large number of microorganisms had become attached to the zeolite.

While the contents of the liquid in the bioreactor were stirred, a 10 ml sample of the suspension present including the zeolite was removed and introduced into a 30 ml test tube having a lid. The sample was followed by 1 ml of 1 M sodium phosphate buffer solution mixed with sodium dihydrogenphosphate and disodium hydrogenphosphate to maintain the pH of the suspension in the test tube at about 4.6. Then 40 mg of cellulase (trade name Meiji Cellulase TP; manufactured by Meiji Seika K.K.) which had been dissolved in distilled water and filtered in the same way as in Example 1 were introduced into the test tube which was then maintained with agitation for 2 hours in a thermoregulated room at 40°C. The resulting suspension was dyed and the number of individual microorganisms was counted. The bacteria were found to be present as individuals and not in groups, and about 5 x 10⁸ of bacteria were found to be present in 1 ml of the sample, giving a total of 1 x 10¹⁰ for the bacteria in the reactor.

### COMPARATIVE EXAMPLE 3

### Repetition in the absence of cellulase

A second 10 ml sample of the suspension in the bioreactor of Example 6 was treated as described in that Example except that the cellulase was not added. Microscopic observation revealed that the bacteria were present in groups, and that about 8 x 10⁶ bacteria in 1 ml of sample had been collected which was only about 2% of the number in Example 6.

### COMPARATIVE EXAMPLE 4

### Repetition with prolonged enzyme treatment

A third 10 ml sample was removed from the bioreactor of Example 6 and treated with cellulase as described except that agitation in a thermoregulated room at 40°C was continued for 13 hours. Thereafter the procedure was as described in Example 6. Microscopic observation revealed that the bacteria were present in groups and that about 4 x 10³ bacteria per 1ml of sample had been collected which was only 0.008% of the number observed in Example 6.

### EXAMPLE 7

### Use of a sucrose-containing medium

3 g of sucrose were introduced into a test tube of volume 5 ml which contained 1 ml of a soil suspension as described in Example 1. The test tube was maintained in a thermoregulated room at 40°C under agitation which was maintained until the supernatant liquor was saturated. The specific gravity of the liquor was 1.3 or above. Thereafter an aqueous solution of sucrose having a specific gravity of 1.3 was added to the solution so as to bring its volume to about 4 ml with thorough stirring. The test tube was then centrifuged at 50000 rpm for 2 hours, after which 3 ml of the supernatant sucrose-saturated liquor was collected and diluted with about 20 ml of distilled water. The diluted solution was centrifuged at 10000 rpm for 10 minutes to precipitate microorganisms present. The collected precipitate was resuspended in 50 ml of distilled water to form a concentrated liquid containing soil microorganisms. This concentrated liquid was fixed with 50 ml of 4% formalin and stained with ethidiumbromide which emits fluorescent light on irradiation with ultraviolet radiation. After the solution had been left for one hour it was stirred thoroughly and diluted suitably (e.g. 100 times). A sample was then observed in a fluorescence microscope from which it was apparent that the microorganisms had been suspended as individuals without grouping into flocks and that there were no soil particles present. The number of individual microorganisms was counted with the result that 3 x 10⁸ of bacteria had been found to be collectable from 1 g (net weight) of the soil. Fig. 1 is a photomicrograph of the liquid sample in which there can be distinguished microorganisms A which because of the high density of liquid had not become collapsed. Apart from the microorganisms, there were found to be present only a few microparticles of size about 0.1 µm, identified by the reference letter B.

### EXAMPLE 8

### Isolation of microorganisms from soil by centrifugation and separation of DNA therefrom

1 ml of the same soil suspension as in Example 1 was introduced into a test tube followed by 0.5 ml of a filtered enzyme solution which contained 40 mg per ml of the above-mentioned cellulase and 100 mg (1000 U) of the protease. The enzyme-containing solution was maintained in a thermoregulated room at 40°C for 2 hours with agitation, after which sucrose was introduced into the mixture in the same manner as in Example 7 to bring the specific gravity to 1.3. The resulting mixture was centrifuged and the supernatant liquor was collected as in Example 1, after which the supernatant was diluted with distilled water and centrifuged to collect the precipitated microorganism. The precipitate was resuspended in 500 µl of 1% SDS solution which was the maintained at 70°C for 1 hour to bacterialise the microorganisms. DNA was extracted from the resulting liquid by means of chloroform, after which the DNA was precipitated from the chloroform by ethenol and collected. The collected DNA was dissolved in distilled water to form a solution containing the DNA present in the soil microorganisms. This DNA solution was subjected to electrophoresis in an agarose gel and about 5000 ng of DNA which had a length 20 kbp or more was collected.

The collected DNA was treated with BamHI and EcoRI which are typical restriction enzymes and maintained at 37°C for 2 hours, after which the solution was subjected to electrophoresis. It was found that the length of the DNA had been reduced to about 5-10 kbp, indicating that the restriction enzymes had successfully digested the DNA. The conditions in the digestion step are as set out in Table 1 below:

**TABLE 1**

| Conditions for digesting the DNA | |
|---|---|
| Collected DNA aqueous solution | 2µl |
| BamHI/EcoRI | 1µl |
| H. Buffer solution for restriction enzyme | 1µl |
| Distilled water | 6µl |
| | 10µl |

It is therefore apparent that DNA extracted from the collected soil microorganisms and refined by centrifugation and selection of specific weight was sufficiently pure for digestion by restriction enzymes without any refining steps.

### EXAMPLE 9

### Electrophoresis followed by DNA separation

Six test tubes having lids were each filled with 6 ml of the soil suspension of Example 1, followed by the previously described filtered solution of cellulase (Meiji Cellulase TP). The test tubes were maintained in a thermoregulated room at 40°C for 2 hours, after which the soil suspension was introduced into a 250 ml electrophoresis tank (See Figure 2) containing about 200 ml of buffer solution having the composition shown in Table 2. Electrophoresis was then carried out by applying 100 V (about 50 mA) of direct voltage between platinum electrodes.

**TABLE 2**

| Composition of the buffer solution for electrophoresis | |
|---|---|
| Tris | 4.08g |
| EDTA.2Na | 0.74g |
| Acetic Acid | 1.14ml |
| H₂O | 1000ml |

4 hours later it was apparent that the soil particles had sufficiently precipitated and the supernatant liquor had become transparent. 10 ml of the buffer solution near the positive platinum electrode was collected by a pipette. The collected sample was centrifuged and precipitated microorganisms were observed at the bottom of the centrifuged tube. This precipitate was collected and introduced into a microtube of volume 1.5 ml, after which a solution of the soil microorganism DNA was made in the same manner as in Example 8. The DNA solution was subjected to electrophoresis using agrose gel, after which about 150 ng of DNA which had 20 KbP or more length was collected. The DNA was digested with BamHI and EcORI in the same manner as in Example 8 and then subjected to electrophoresis, as a result of which it was found that the DNA had been digested and its length had been shortened to about 5-20 KbP. It is therefore apparent that the microorganisms can be refined by electrophoresis, after which they can be collected and the DNA extracted therefrom to give a sample having sufficient purity for digestion by restriction enzymes without additional refining.

### COMPARATIVE EXAMPLE 5

### Repetition without DNA refining step

1 ml of the same soil suspension as in Example 1 was introduced into a test tube having a lid followed by cellulase (Meiji Cellulase TP), after which the enzyme-containing solution was maintained in a thermoregulated room at 40°C with agitation. The test tube was then centrifuged at 5000 rpm for 30 seconds to precipitate soil particles and 1.5 ml of the supernatant liquor was collected and again centrifuged at 1500 rpm for 5 minutes. The resulting precipitate was collected, introduced into a 1.5 ml volume microtube, and a soil microorganism DNA solution was formed in the same manner as in Example 8. This DNA solution was subjected to electrophoresis on agarose gel and about 150ng of DNA having a length of 20KbP or above was collected. Attempted digestion of the DNA with BamHI and EcoRI was carried out in the same manner as described in Example 8 and the digested solution was subjected to electrophoresis. However, it was found that the digested DNA had almost the same length as the DNA before digestion, indicating that the restriction enzymes had not worked. It is apparent from this experiment that even where a microorganism has been separated into free individuals using an enzyme, the collected DNA will not be digested by restriction enzyme unless impurities have been sufficiently removed in the refining step, e.g. by extraction into chloroform and then re-precipitation.

### EXAMPLE 10

### Use of alternative enzymes

The procedure of Example 8 was repeated using the following enzyme preparations:
(a) 500 µl (25000 U) of a liquid enzyme preparation containing mainly amylase (trade name: Termamyl; manufactured by Novo Nordisk Bioindustry);
(b) The above mentioned amylase in the amount indicated together with 50 mg of cellulase;
(c) 50 mg of enzyme powder mainly containing protease (protease activity 500 U) available under the trade name Protease A Amano manufactured by Amano Pharmaceutical Co., Ltd.;
(d) A mixture of protease in the above described amount and cellulase in the above described amount;
(e) 100 mg of an enzyme powder containing mainly glucoronidase sold under the trade name Abalone Aceton Powder; manufactured by Sigma Chemical Co.; and
(f) The above mentioned amount of glucoronidase and the above mentioned amount of amylase and/or protease.

The same results as in Example 8 were obtained, and in particular the number of separated individual microorganisms was high in the examples where the mixtures of enzymes were used. On the other hand, if the experiments were repeated in the absence of enzymes or using deactivated enzymes, the number of separate individual microorganisms was only 5 to 10% of those reported in the previous examples.

In a further series of experiments, the same enzymes or mixtures of enzymes were used with the microorganisms attached to zeolite obtained by the method described in Example 6, and the experiments were conducted both with enzyme present and with enzyme omitted. As a result, when the enzyme was present it was found that individual microorganisms had been extracted and that their number was about 100-200 times the number extracted when no enzyme was used. Where the enzyme was present the individual microorganisms could readily be separated and collected.

### EXAMPLE 11

### Detection of a single type of bacteria by antibody labelling and fluorescence counting of labelled bacteria

A mixed solution of sodium phosphate buffer, soil and enzyme was made as described in Example 1, after which the solution was shaken for 2 hours at 40°C in a thermoregulated room. Then 3g of sucrose was added to the solution which was stirred until the supernatant liquor had become saturated. The liquor had a specific gravity of 1.3 or above. Then an aqueous solution of sucrose having a specific gravity of 1.3 was added to the original solution to bring its volume to about 4 ml, with thorough stirring. The test tube containing solution was centrifuged at 50000 rpm for 2 hours after which a 3 ml of supernatant sucrose-saturated liquor was collected and diluted with 20 ml of distilled water. The diluted solution was centrifuged at 10000 rpm for 10 minutes and the resulting precipitate was collected. The precipitate was suspended in 500 µl of balanced saline solution. A suspension of soil microorganisms was made by resuspending the resulting precipitate in 500 µl of balanced saline solution which contained 1% of bovine serum albumin buffered with phosphoric acid. The suspension was then treated with 0.5 ml of a solution containing 0.2 mg/ml of an anti P. Cepacia monoclonal antibody tagged with FITC. The solution was maintained for 1 hour at 0°C to permit the antigen-antibody reaction to take place. The anti P. Cepacia monoclonal antibody was produced by selecting a mouse of the KK01 strain (FERM BP-4235), immunising the mouse by conventional methods, and fusing antibody-producing mouse cells again by convention methods [(see: Japan J. Med. Sei. Biolo., 37, pp 151-159 (1984)]. The resulting monoclonal antibody was tagged with FITC (maximum fluorescent wavelength is 530 nm) by adding FITC and the antibody to a carbonic acid buffer solution at a pH of 9.5, permitting them to react for 1 hour and then removing free FITC using a column of Sephadex G-25 (Registered Trade Mark) (see "MENEKISEIKAGAKUKENKYUHO" [Immunity Biochemical Experimental Method], 1986, [edited by NIPPON SEIKAGAKUKAI (Society of Biochemistry), published by Tokyo Kagaku Dojinsha K.K.] pp 103-105). The bonding ratio of fluorescent dye to antibody protein (F/P ratio was 4.2).

After the antibody reaction, 1 ml of the suspension was centrifuged for 10 minutes at 4000 rpm after which the supernatant liquor was removed. Then 1 ml phosphate buffered saline solution containing 25 µg/ml of ethidium bromide was added and the mixture was allowed to stand for 2 hours at room temperature. After this time the liquid was centrifuged and the supernatant was removed.

This cleaning step was repeated 3 times, after which the precipitated microorganisms were resuspended in 5 ml of phosphate buffered saline and diluted 10 times to produce a sample for measurement. The sample was subjected to supersonic waves for a period of 5 seconds to disperse particles therein, after which the number of bacteria in the sample was counted by means of a FACS-CAN counter made by Beckton & Dickinson Co., Ltd. The way the machine is set up will be apparent from Figs. 3(a) to 3(c). Fig. 3(a) is a graph showing forward scatter against side scatter. Fig. 3(b) is a histogram of fluorescent light FL1 at 530 nm corresponding to the fluorescence of FITC in the labelled antibody. FL3 corresponds to the fluorescence of the ethidium bromide used as a stain for the bacteria and is at 650 nm. The sample was allowed to flow through the counter for 1 minute at a rate of 12 µl per minute, and observations were made when particles passed the counter. The signals FL3 come both from stained bacteria and from impurities which do not contain nucleic acid such as proteins, lipids or microparticles such as dust present. To minimise the background, a trigger or gate was set in the channel which corresponds to the fluorescent light FL3 of the ethidium bromide stain, and this appears as an arrow mark in Fig. 3(c). At the same time, the channel sensitive to the FL1 fluorescence at 530 nm detects the separate fluorescent light corresponding to FITC, and this signal is gated with a desired width of signal level as is shown in Fig. 3(b). The width of the gate is arranged to detect fluorescent light of the strength coming from the FITC labelled bacteria. Pulses produced by the counter during the 1 minute timing interval represent the total number of bacteria found. As a result it was determined that the total number of bacteria was 3.7 x 10⁸, and the estimated number of P. Cepacia was 4.1 x 10⁶, both in 1 gm of soil.

### EXAMPLE 12

### Detecting of two kinds of bacteria by antibody labelling and fluoresence counting

Six test tubes with lids were provided and 6 ml of the same soil suspension as in Example 10 was introduced into each of the 6 test tubes. The cellulase enzyme solution of Example 1 was introduced into each test tube, and they were maintained with agitation for 2 hours in a room thermoregulated to 40°C as previously described. The resulting soil suspension was introduced into a 250 ml electrophoresis tank containing 200 ml of buffer solution having the composition shown in Table 2 above, and electrophoresis was carried out by applying a 100 volts (about 50 mA) of DC across platinum electrodes. After 4 hours it was apparent that the soil particles had sufficiently precipitated and the supernatant liquor was transparent. 10 ml of buffer solution adjacent to the positive platinum electrode was collected by a pipette and the sample was centrifuged to give a precipitate which was then resuspended in 500 µl of liquid containing 1% of phosphate buffered saline and 1% bovine serum albumin.

An anti P. putida monoclonal antibody tagged with PE was provided by mixing PE (manufactured by PolySciences Company) and with N-succinimide-3-(2-pyridyldithio) propionic acid thiol groups and with the anti P. putida monoclonal antibody with which the thiolated PE couples. The F/P ratio was 1.7. The anti P. Cepacia monoclonal antibody used as starting material was made by the same procedure as described in Example 11.

0.5 ml solution containing anti P. Cepacia monoclonal antibody tagged with FITC (0.2 g/ml as used in Example 11 and 0.5 ml of a solution containing anti P.putida monoclonal antibody tagged with PE (maximum fluorescent wavelength 580 nm; 0.2 mg/ml) was added to the suspension and an antigen-antibody reaction was allowed to take place for 1 hour at 0°C. Then the resulting liquid was centrifuged for 10 minutes at 4000 rpm per minute and the supernatant liquor was removed. 1 ml of PBS solution containing ethidium bromide 25 µg/ml was added to the supernatant and reaction was allowed to take place for 2 hours at room temperature, after which the supernatant liquor was removed. The precipitated microorganisms were washed with 5 ml of PBS and centrifuged 3 times, after which the cleaned precipitate was resuspended in 5 ml of PBS and diluted 10 times to provide a sample for measurement. The solid materials in the sample were dispersed by supplying supersonic waves for 5 seconds, after which the number of bacteria in the sample was counted by a FAS-CAN counter using 3 channels one of which counts fluorescence from P. Cepacia bacteria labelled with FITC (FL1:530 ± 15 nm), a second of which counts P.putida bacteria labelled by PE (FL2:585 ± 21 nm) and the third of which (FL3) detects fluorescent light from ethidium bromide (FL3:650 nm or above). The sample was allowed to flow at a rate of 12 µl/min. for 1 minute while pulses in the respective channels FL1 and FL2 were counted to provide an estimate of the number of the two different types of bacteria detected. By this means it was determined that a total number of bacteria recovered was 3.5 x 10⁸ and that the population of P. Cepacia was 4.0 x 10⁶ whilst that of P. putida was 6.9 x 10⁶ each in 1 gm of soil.

### COMPARATIVE EXAMPLE 6

### Repetition without enzymes

10 g of the soil sample was processed in the same way as in Example 1 to produce a soil suspension, after which the suspension was filtered using a filter having holes of diameter 15 µm to remove large suspended soil particles. There was no enzyme treatment, or separation and collection of microorganisms. 50 µl of the filtrate was centrifuged at 10,000 rpm for ten minutes and the precipitate was collected. Phosphate buffered saline (PBS) containing 1% bovine serum albumin (BSA) was added to the precipitate to give a volume of 500 µl, after which the precipitate was resuspended to form a soil-containing liquid. The liquid was treated with anti P. Cepacia antibody and the two were allowed to react. A sample stained with ethidium bromide was produced. Thereafter a measurement procedure was carried out, all of the steps being as described in Example 10. As a result, the total number of bacteria recovered was found to be 4.1 x 10⁶ and 4.4 x 10⁴ of P. Cepacia were detected. However, the amount of bacteria detected was about 100th of that in Example 1 which is believed to be because almost all of them remained adhered to the soil particles, or were cohered to one another and were therefore not collected.

The above Examples show that an enzyme can be used to decompose insoluble organic polymer secreted by microorganisms, which polymer may cause the microorganism to adhere either to a carrier or to another microorganism. Decomposition of the polymer which acts as a bonding agent enables individual microorganisms to be separated and collected from a suspension or from a liquid in a bioreactor very easily in a short time with high yields and high purity without the need for their particular culturing conditions to be known or for specific culturing steps to be carried out. The polymer bonding compound which holds the microorganism to the carrier or holds the microorganisms together may comprise a range of polysaccharides or insoluble proteins depending on the species of microorganism. The separation efficiency can be improved by selecting an enzyme, or mixture of enzymes, which can decompose the particular bonding compounds in question. Such enzymes may be selected from cellulose, hemicellulose, glucuronidase, amilase, protease and pectinase. The process of the invention can be used on soil or on material recovered from a bioreactor for example. It enables useful microorganisms adhered to a carrier, or other microorganisms which may exist in the soil or on the bioreactor, to be separated into individual microorganisms which can be collected.

## Claims

1. A process for obtaining individual microorganisms from a microorganic system in which individual microorganisms are adhered to a carrier or to other individual microorganisms with a natural adhesive secreted from the individual microorganisms, characterised in that the process comprises the step of treating the microorganic system with at least one enzyme selected from the group consisting of enzymes which decompose polysaccharides, enzymes which decompose proteins, and enzymes which decompose pectin thereby separating the individual microorganisms from the carrier or other individual microorganisms.

2. The process of claim 1, wherein the enzyme comprises cellulase, hemicellulase, glucuronidase, amylase, protease or pectinase or a mixture thereof.

3. The process of claim 1 or claim 2, wherein the microorganic system comprises a soil suspension, a suspension of activated sludge or a suspension of mud from rivers, lakes or seas.

4. The process of claim 1 or claim 2, wherein the microorganic system comprises reactor liquid which contains a carrier for supporting microorganisms in a bioreactor.

5. The process of any preceding claim, wherein the separated microorganisms are refined and collected.

6. The process of claim 5, wherein the micoorganisms are refined and collected by forming a suspension containing the separated microorganisms and having a similar specific gravity to that of the microorganisms; and centrifuging the suspension and separating the suspension into a supernatant liquor containing the microorganisms and precipitate by using the difference in specific gravity between the dispersive medium and particles to be separated.

7. The process of claim 6, wherein the procedure for refining and collecting the microorganism comprises the steps of:
adjusting the specific gravity of the enzyme treated suspension to be the same as the specific gravity of the microorganism, or to be higher than the specific gravity of the microorganism, or to be lower than the specific gravity of the carrier;
centrifuging the suspension whose specific gravity has been adjusted so as to precipitate the carrier and to obtain a supernatant liquor in which the microorganisms are suspended; and
collecting the microorganism from the supernatant liquor.

8. The process of claim 7, wherein the specific gravity of the suspension is adjusted to be in the range 1.2 to 1.5.

9. The process of claim 7 or 8, wherein the specific gravity of the suspension is adjusted by adding a solute.

10. The process of claim 9, wherein the solute comprises sucrose.

11. The process of claim 9, wherein the solute comprises cesium chloride.

12. A process according to claim 5, wherein the microorganism is refined by means of its electrophoretic mobility and is collected.

13. A process according to claim 12, in which the step of refining and collecting the microorganism using its electrophoretic mobility comprises:
applying a DC voltage to a suspension of the microorganism after it has been treated with the enzyme; and
collecting liquid containing the microorganism which has been moved adjacent to the positive and/or the negative electrode.

14. A process for counting a number of individual microorganisms present in a sample, comprising the steps of collecting liquid which contains individual microorganisms separated by the process of any of claims 1-4, and counting the number of microorganisms present in said liquid.

15. The process of claim 14, wherein the microorganisms have been separated, refined and collected according to any of claims 6-13.

16. A process according to claim 15, wherein the counting is carried out using the plate dilution method or the dyeing method.

17. A process for collecting a nucleic acid which comprises extracting and collecting nucleic acid of a microorganism from a liquid containing the microorganism which was collected by the process defined in any of claims 6-13.

18. A process for counting the distribution of a particular microorganism in a microorganic system including a plurality of kinds of microorganism, in which there is at least one specific microorganism adhered to particles present in the system or cohered with other microorganisms, said process comprising:
obtaining a suspension of separated microorganisms by the process claimed in any of claims 1-13 and collecting the separated microorganisms from the suspension;
bonding to the specific microorganism an antibody tagged with a fluorescent dye;
bonding to the microorganisms present a fluorescent compound whose fluorescence maximum wavelength is different from the wavelength of the fluorescent dye; and
measuring the fluorescence emitted by the fluorescent dye and the fluorescent compound.

19. A process of claim 18, wherein the fluorescence from the fluorescent dye and the fluorescent compound are measured by:
forming a flow of the suspension and detecting in the flow the fluorescence from the dye and the fluorescence from the compound for each particle passing through the flow; and
processing the detected data statistically to derive said distribution information.

20. The process of claim 20, wherein a trigger or gate has been set responsive to fluorescence from said fluorescent compound so as to pass the signal only when it exceeds a threshold.

21. The process of any of claims 18, 19 or 20, further comprising the step of supplying vibrational energy or shear energy to the suspension before the fluoresence from the fluorescent dye and the fluorescent compound are measured so as to separate groups of microorganisms cohered by the antibody.

22. A process of any of claims 18-21, wherein the maximum fluorescent wavelength of the fluorescent dye is separated by 20 nm or more from the maximum fluorescent wavelength of the fluorescent compound.

23. The process of any of claims 18-22, wherein the fluorescence compound is a dye which bonds to nucleic acid of microorganisms.

24. The process of any of claims 18-23, wherein the antibody tagging step comprises bonding a plurality of different antibodies tagged with fluorescent dye to a plurality of different types of microorganisms.

25. A process according to any of claims 18-24, wherein the antibody bonding is direct.

26. A process according to any of claims 18-24, wherein the antibody bonding is indirect.

27. A process according to any of claims 18-26, wherein the microorganic system comprises soil, activated sludge, or marine mud from fresh, tidal or salt water.

28. A process according to any of claims 18-27, wherein the microorganic system comprises a reaction mixture from a bioreactor containing in suspension a carrier on which a microorganism is supported.

## Patentansprüche

1. Verfahren zur Gewinnung von einzelnen Mikroorganismen aus einem mikroorganischen System, in dem einzelne Mikroorganismen mittels eines natürlichen Klebstoffs, der von den einzelnen Mikroorganismen abgesondert wird, an einem Träger oder an andere einzelne Mikroorganismen gebunden sind,
**dadurch gekennzeichnet, daß**
das Verfahren den Schritt der Behandlung des mikroorganischen Systems mit mindestens einem Enzym umfaßt, das aus der Gruppe ausgewählt ist, die aus Enzymen, die Polysaccharide abbauen, Enzymen, die Proteine abbauen, und Enzymen, die Pectin abbauen, besteht, wodurch die einzelnen Mikroorganismen von dem Träger oder den anderen einzelnen Mikroorganismen abgetrennt werden.

2. Verfahren nach Anspruch 1,
**dadurch gekennzeichnet, daß**
das Enzym Cellulase, Hemicellulase, Glucuronidase, Amylase, Protease oder Pectinase oder eine Mischung davon umfaßt.

3. Verfahren nach Anspruch 1 oder Anspruch 2,
**dadurch gekennzeichnet, daß**
das mikroorganische System eine Bodensuspension, eine Suspension von belebtem Schlamm oder eine Suspension von Schlamm aus Flüssen, Seen oder Meeren umfaßt.

4. Verfahren nach Anspruch 1 oder Anspruch 2,
**dadurch gekennzeichnet, daß**
das mikroorganische System eine Reaktorflüssigkeit, die einen Träger zum Tragen der Mikroorganismen enthält, in einem Bioreaktor umfaßt.

5. Verfahren nach einem der vorstehenden Ansprüche,
**dadurch gekennzeichnet, daß**
die getrennten Mikroorganismen gereinigt und gewonnen werden.

6. Verfahren nach Anspruch 5,
**dadurch gekennzeichnet, daß**
die Mikroorganismen durch die Bildung einer Suspension, die die getrennten Mikroorganismen enthält und ein ähnliches spezifisches Gewicht wie das der Mikroorganismen aufweist, und das Zentrifugieren der Suspension und das Trennen der Suspension durch die Nutzung des Unterschieds des spezifischen Gewichts zwischen dem Dispersionsmedium und den zu trennenden Teilchen in eine überstehende Flüssigkeit, die die Mikroorganismen enthält, und einen Bodenkörper gereinigt und gewonnen werden.

7. Verfahren nach Anspruch 6,
**dadurch gekennzeichnet, daß**
der Prozeß zum Reinigen und Gewinnen des Mikroorganismus die nachstehenden Schritte umfaßt:
Einstellen des spezifischen Gewichts der enzym-behandelten Suspension auf den gleichen Wert, den das spezifische Gewicht des Mikroorganismus aufweist, oder auf einen höheren Wert als den, den das spezifische Gewicht des Mikroorganismus aufweist, oder auf einen kleineren Wert als den, den das spezifische Gewicht des Trägers aufweist;
Zentrifugieren der Suspension, deren spezifisches Gewicht so eingestellt wurde, daß der Träger abgeschieden und eine überstehende Flüssigkeit erhalten wird, in der die Mikroorganismen suspendiert sind; und
Gewinnen des Mikroorganismus aus der überstehenden Flüssigkeit.

8. Verfahren nach Anspruch 7,
**dadurch gekennzeichnet, daß**
das spezifische Gewicht der Suspension so eingestellt wird, daß es in einem Bereich von 1,2 bis 1,5 liegt.

9. Verfahren nach Anspruch 7 oder Anspruch 8,
**dadurch gekennzeichnet, daß**
das spezifische Gewicht der Suspension durch Hinzufügen eines gelösten Stoffes eingestellt wird.

10. Verfahren nach Anspruch 9,
**dadurch gekennzeichnet, daß**
der gelöste Stoff Saccharose umfaßt.

11. Verfahren nach Anspruch 9,
**dadurch gekennzeichnet, daß**
der gelöste Stoff Cäsiumchlorid umfaßt.

12. Verfahren nach Anspruch 5,
**dadurch gekennzeichnet, daß**
der Mikroorganismus mittels seiner elektrophoretischen Beweglichkeit gereinigt wird und gewonnen wird.

13. Verfahren nach Anspruch 12,
**dadurch gekennzeichnet, daß**
der Schritt der Reinigung und Gewinnung des Mikroorganismus unter Nutzung seiner elektrophoretischen Beweglichkeit die nachstehenden Schritte umfaßt:
Anlegen einer Gleichspannung an die Suspension des Mikroorganismus, nachdem sie mit dem Enzym behandelt worden ist; und
Gewinnen der Flüssigkeit, die den Mikroorganismus enthält, der in die Nähe der positiven und/oder der negativen Elektrode bewegt worden ist.

14. Verfahren zum Zählen der Anzahl der in einer Probe vorhandenen einzelnen Mikroorganismen, das die Schritte der Gewinnung einer Flüssigkeit, die die einzelnen Mikroorganismen enthält, die durch das Verfahren nach einem der Ansprüche 1 bis 4 getrennt wurden, und des Zählens der Anzahl der in der Flüssigkeit vorhandenen Mikroorganismen umfaßt.

15. Verfahren nach Anspruch 14, in dem die Mikroorganismen nach einem der Ansprüche 6 bis 13 getrennt, gereinigt und gewonnen wurden.

16. Verfahren nach Anspruch 15, in dem das Zählen unter Anwendung eines Plattenverdünnungsverfahrens oder eines Färbeverfahrens erfolgt.

17. Verfahren zur Gewinnung einer Nucleinsäure, das das Extrahieren und das Gewinnen der Nucleinsäure eines Mikroorganismus aus einer Flüssigkeit umfaßt, die den Mikroorganismus enthält, der mittels des Verfahrens, wie es in einem der Ansprüche 6 bis 13 definiert ist, gewonnen wurde.

18. Verfahren zum Zählen der Verteilung eines bestimmten Mikroorganismus in einem mikroorganischen System, das eine Vielzahl von Arten von Mikroorganismen einschließt, in der mindestens ein bestimmter Mikroorganismus vorhanden ist, der an Teilchen, die in dem System auftreten, gebunden ist, oder mit anderen Mikroorganismen zusammenklebt, wobei das Verfahren die nachstehenden Schritte umfaßt:
Erhalt einer Suspension getrennter Mikroorganismen durch das Verfahren nach einem der Ansprüche 1 bis 13 und Gewinnen der getrennten Mikroorganismen aus der Suspension;
Binden eines Antikörpers, der mit einem fluoreszierten Farbstoff markiert ist, an den bestimmten Mikroorganismus;
Binden einer fluoreszierenden Verbindung, deren maximale Fluoreszenz-Wellenlänge sich von der Wellenlänge des fluoreszierenden Farbstoffs unterscheidet, an die vorhandenen Mikroorganismen; und
Messen der Fluoreszenz, die von dem fluoreszierenden Farbstoff und der fluoreszierenden Verbindung emittiert wird.

19. Verfahren nach Anspruch 18, in dem die Fluoreszenz von dem fluoreszierenden Farbstoff und der fluoreszierenden Verbindung mittels der nachstehenden Maßnahmen gemessen werden:
Bildung eines Suspensionsstroms und Ermittlung der Fluoreszenz von dem Farbstoff und der Fluoreszenz von der Verbindung in dem Strom für jedes Teilchen, das den Strom passiert, und
statistisches Verarbeiten der ermittelten Daten, um die Verteilungsinformation zu gewinnen.

20. Verfahren nach Anspruch 20, in dem ein Auslöser oder Schaltelement so eingestellt wurde, daß er/es auf die Fluoreszenz von der fluoreszierenden Verbindung anspricht, um das Signal nur dann durchzulassen, wenn es eine bestimmte Schwelle überschreitet.

21. Verfahren nach Anspruch 18, Anspruch 19 oder Anspruch 20, das ferner den Schritt der Zufuhr von Schwingungsenergie oder Scherenergie zu der Suspension umfaßt, bevor die Fluoreszenz von dem fluoreszierenden Farbstoff und der fluoreszierenden Verbindung gemessen werden, um Gruppen von Mikroorganismen, die durch den Antikörper zusammenkleben, zu trennen.

22. Verfahren nach einem der Ansprüche 18 bis 21, in dem die maximale Fluoreszenz-Wellenlänge des fluoreszierenden Farbstoffes um 20 nm oder mehr von der maximalen Fluoreszenz-Wellenlänge der fluoreszierenden Verbindung getrennt ist.

23. Verfahren nach einem der Ansprüche 18 bis 22, in dem die Fluoreszenzverbindung ein Farbstoff ist, der eine Bindung mit der Nucleinsäure der Mikroorganismen eingeht.

24. Verfahren nach einem der Ansprüche 18 bis 23, in dem der Schritt der Markierung mit dem Antikörper das Binden einer Vielzahl verschiedener Antikörper, die mit einem fluoreszierenden Farbstoff markiert sind, an eine Vielzahl verschiedener Arten von Mikroorganismen umfaßt.

25. Verfahren nach einem der Ansprüche 18 bis 24, in dem die Antikörperbindung eine direkte ist.

26. Verfahren nach einem der Ansprüche 18 bis 24, in dem die Antikörperbindung eine indirekte ist.

27. Verfahren nach einem der Ansprüche 18 bis 26, in dem das mikroorganische System Boden, belebten Schlamm oder Meerschlamm aus frischem Wasser, Gezeiten- oder Salzwasser umfaßt.

28. Verfahren nach einem der Ansprüche 18 bis 27, in dem das mikroorganische System eine Reaktionsmischung aus einem Bioreaktor umfaßt, der einen Träger in Suspension enthält, der einen Mikroorganismus trägt.

## Revendications

1. Procédé d'obtention de micro-organismes individuels à partir d'un système de micro-organismes dans lequel des micro-organismes individuels adhèrent à un support ou à d'autres micro-organismes individuels au moyen d'un adhésif naturel sécrété par les micro-organismes individuels, caractérisé en ce que ledit procédé comprend l'étape consistant à traiter le système de micro-organismes avec au moins une enzyme choisie dans l'ensemble constitué par des enzymes qui décomposent les polysaccharides, des enzymes qui décomposent les protéines et des enzymes qui décomposent la pectine, séparant ainsi les micro-organismes individuels à partir du support ou d'autres micro-organismes individuels.

2. Procédé selon la revendication 1, dans lequel l'enzyme comprend une cellulase, une hémicellulase, une glucuronidase, une amylase, une protéase ou une pectinase ou un mélange de celles-ci.

3. Procédé selon la revendication 1 ou la revendication 2, dans lequel le système de micro-organismes comprend une suspension de terre, une suspension de boue activée ou une suspension de boue provenant des rivières, des lacs ou des mers.

4. Procédé selon la revendication 1 ou la revendication 2, dans lequel le système de micro-organismes comprend un réactif liquide qui contient un support pour supporter les micro-organismes dans un bioréacteur.

5. Procédé selon l'une quelconque des revendications précédentes, dans lequel les micro-organismes séparés sont purifiés et recueillis.

6. Procédé selon la revendication 5, dans lequel on purifie et on recueille les micro-organismes en formant une suspension contenant les micro-organismes séparés et ayant un poids volumique analogue à celui des micro-organismes, et en centrifugeant la suspension et en séparant celle-ci en une solution surnageante contenant les micro-organismes et en un précipité en utilisant la différence de poids volumique entre le milieu dispersif et les particules à séparer.

7. Procédé selon la revendication 6, dans lequel le mode opératoire pour purifier et recueillir le microorganisme comprend les étapes consistant à :
ajuster le poids volumique de la suspension traitée avec une enzyme jusqu'à ce qu'il soit égal au poids volumique du micro-organisme, ou jusqu'à ce qu'il soit supérieur au poids volumique du micro-organisme, ou jusqu'à ce qu'il soit inférieur au poids volumique du support ;
centrifuger la suspension dont le poids volumique a été ajusté de façon à faire précipiter le support et à obtenir une solution surnageante dans laquelle les micro-organismes sont en suspension ; et
recueillir le micro-organisme à partir de la solution surnageante.

8. Procédé selon la revendication 7, dans lequel on ajuste le poids volumique de la suspension entre 1,2 et 1,5.

9. Procédé selon la revendication 7 ou 8, dans lequel on ajuste le poids volumique de la suspension en ajoutant un soluté.

10. Procédé selon la revendication 9, dans lequel le soluté comprend du saccharose.

11. Procédé selon la revendication 9, dans lequel le soluté comprend du chlorure de césium.

12. Procédé selon la revendication 5, dans lequel on purifie le micro-organisme au moyen de sa mobilité électrophorétique et on le recueille.

13. Procédé selon la revendication 12, dans lequel l'étape de purification et de collecte du micro-organisme en utilisant sa mobilité électrophorétique comprend les étapes consistant à :
appliquer une tension continue à une suspension du micro-organisme après l'avoir traitée avec l'enzyme ; et
recueillir le liquide contenant le micro-organisme qui a été déplacé de façon contigué à l'électrode positive et/ou à l'électrode négative.

14. Procédé pour compter un nombre de micro-organismes individuels présents dans un échantillon, comprenant les étapes consistant à recueillir un liquide qui contient des micro-organismes individuels séparés au moyen du procédé selon l'une quelconque des revendications 1 à 4 et à compter le nombre de micro-organismes présents dans ledit liquide.

15. Procédé selon la revendication 14, dans lequel les micro-organismes sont séparés, purifiés et recueillis selon l'une quelconque des revendications 6 à 13.

16. Procédé selon la revendication 15, dans lequel on effectue le comptage en utilisant le procédé de dilution sur plaque ou le procédé de coloration.

17. Procédé pour recueillir un acide nucléique, comprenant les étapes consistant à extraire et à recueillir un acide nucléique d'un micro-organisme à partir d'un liquide contenant le micro-organisme qu'on a recueilli au moyen du procédé défini dans l'une quelconque des revendications 6 à 13.

18. Procédé de comptage de la distribution d'un micro-organisme spécifique dans un système de micro-organismes comprenant une pluralité de types de micro-organismes, dans lequel au moins un micro-organisme spécifique adhère à des particules présentes dans le système ou adhère à d'autres micro-organismes, ledit procédé comprenant les étapes consistant à :
obtenir une suspension de micro-organismes séparés au moyen du procédé selon l'une quelconque des revendications 1 à 13 et recueillir les micro-organismes séparés à partir de la suspension ;
lier au micro-organisme spécifique un anticorps marqué par un colorant fluorescent ;
lier aux micro-organismes présents un composé fluorescent dont la longueur d'onde maximale de fluorescence est différente de la longueur d'onde du colorant fluorescent ; et
mesurer la fluorescence émise par le colorant fluorescent et par le composé fluorescent.

19. Procédé selon la revendication 18, dans lequel on mesure la fluorescence émise par le colorant fluorescent et par le composé fluorescent en :
formant un flux de la suspension et en détectant dans ce flux la fluorescence émise par le colorant et la fluorescence émise par le composé pour chaque particule traversant le flux ; et
traitant statistiquement les données détectées pour en tirer une information sur ladite distribution.

20. Procédé selon la revendication 19, dans lequel on insère un déclencheur ou une porte responsable de la fluorescence par rapport audit composé de fluorescence de façon à faire passer le signal seulement quand il dépasse une valeur seuil.

21. Procédé selon l'une quelconque des revendications 18, 19 ou 20, comprenant, en outre, l'étape consistant à soumettre la suspension à une énergie vibratoire ou a une énergie de cisaillement avant de mesurer la fluorescence émise par le colorant fluorescent et par le composé fluorescent de façon à séparer des groupes de micro-organismes agglutinés par l'anticorps.

22. Procédé selon l'une quelconque des revendications 18 à 21, dans lequel la longueur d'onde maximale de fluorescence du colorant fluorescent est séparée de la longueur d'onde maximale de fluorescence du composé fluorescent de 20 nm ou plus.

23. Procédé selon l'une quelconque des revendications 18 à 22, dans lequel le composé fluorescent est un colorant qui se lie à un acide nucléique de micro-organismes.

24. Procédé selon l'une quelconque des revendications 18 à 23, dans lequel l'étape de marquage de l'anticorps comprend la liaison d'une pluralité d'anticorps différents marqués par un colorant fluorescent à une pluralité de différents types de micro-organismes.

25. Procédé selon l'une quelconque des revendications 18 à 24, dans lequel la liaison à l'anticorps est directe.

26. Procédé selon l'une quelconque des revendications 18 à 24, dans lequel la liaison à l'anticorps est indirecte.

27. Procédé selon l'une quelconque des revendications 18 à 26, dans lequel le système de micro-organismes comprend de la terre, une boue activée ou de la boue marine provenant d'eau fraîche, d'eau de marée ou d'eau salée.

28. Procédé selon l'une quelconque des revendications 18 à 27, dans lequel le système de micro-organismes comprend un mélange de réaction provenant d'un bioréacteur contenant un support en suspension qui supporte un micro-organisme.
